# EUROPÄISCHE PATENTSCHRIFT

(11) **EP 2 130 565 B1**
(45) Veröffentlichungstag und Bekanntmachung des Hinweises auf die Patenterteilung: **13.11.2013**
(21) Anmeldenummer: 09161703.5
(22) Anmeldetag: 02.06.2009
(51) Int. Cl.: A61M 39/10, F16L 37/23, A61M 39/00

(54) **Kupplung für ein medizinisches Instrument**
Connector for a medical instrument
Connecteur pour un instrument médical

(30) Priorität: 03.06.2008 DE 102008027676
(43) Veröffentlichungstag der Anmeldung: 09.12.2009
(73) Patentinhaber: Karl Storz GmbH & Co. KG, 78532 Tuttlingen (DE)
(72) Erfinder: Doll, Frank, 78607, Talheim (DE); Hahn, Martin, 88605, Boll (DE); Bogenschütz, Jürgen, 88499, Altheim (DE)
(74) Vertreter: Witte, Weller & Partner

(56) Entgegenhaltungen:
- EP-A1- 0 442 310
- US-A- 2 505 093
- US-A- 4 949 745
- US-A1- 2002 149 200
- US-A1- 2008 290 657

## Beschreibung

Die Erfindung betrifft eine Kupplung für ein medizinisches Instrument zum Verbinden zweier Leitungen, mit einem ersten Kupplungsteil und einem zweiten Kupplungsteil, das in das erste Kupplungsteil in Längsrichtung der Kupplungsteile einführbar ist, weiterhin mit am ersten Kupplungsteil angeordneten ersten Verriegelungsmitteln und am zweiten Kupplungsteil angeordneten zweiten Verriegelungsmitteln, wobei die ersten Verriegelungsmittel zwischen einer Verriegelungsstellung, in der sie mit den zweiten Verriegelungsmitteln in Eingriff stehen, und einer Entriegelungsstellung, in der sie mit den zweiten Verriegelungsmitteln außer Eingriff stehen, beweglich sind, und ferner mit am ersten Kupplungsteil angeordneten Arretiermitteln, die zwischen einer Arretierstellung, in der sie die ersten und zweiten Verriegelungsmittel in Eingriff miteinander halten, und einer Entarretierstellung, in der die ersten und zweiten Verriegelungsmittel außer Eingriff bringbar sind, beweglich sind.

Die Erfindung betrifft ferner ein Verfahren zum Herstellen eines ersten Kupplungsteils.

Unter einer Leitung im Sinne der Erfindung werden alle Arten von röhrenförmigen Gebilden, aber auch Leitungen für Elektrizität oder Licht verstanden. Beispiele sind Leitungen für Fluide, also Flüssigkeiten oder Gase, beispielsweise flexible Schläuche oder auch starre Rohrleitungen, Instrumentenkanäle, aber auch elektrische Leitungen und Lichtleiter.

Eine Kupplung mit den eingangs genannten konstruktiven Merkmalen ist aus dem deutschen Gebrauchsmuster DE 79 09 593 U1 bekannt.

US 4,949,745 beschreibt einen Anschluss zur Verwendung in einer kontaminierten Umgebung. Der aus diesem Dokument bekannte Anschluss weist ein erstes und ein zweites Kupplungsteil auf, die gekoppelt werden, um einen Flussweg für ein Fluid durch den Anschluss zu schaffen. Oberflächen, die kontaminiert sein können und die innerhalb des Wegs des Fluids liegen, sind im geschlossenen Zustand so innerhalb der Kupplung angeordnet, dass möglicherweise vorliegende Kontaminanten zwischen verschiedenen Dichtungen eingeschlossen sind. Ferner ist hierbei ein System zum Spülen des Anschlusses vorgesehen.

US 2002/0149200 beschreibt eine Buchse für ein Dialysegerät, wobei ein federbelasteter Halter vorgesehen ist, um eine Kugel nach außen zu drücken. Der federbeaufschlagte Halter ist zwischen einem äußeren Mantel, der an der äußeren Oberfläche des Kupplungskörpers angeordnet ist, und der äußeren Oberfläche des Kupplungsteils angeordnet, wobei dieser vor und zurück bewegt werden kann.

Aus EP 0 442 310 geht ein Stecker, der als Nippel bezeichnet wird, hervor, der dazu ausgelegt ist, mit verschiedenen weiteren Kupplungsteilen verbunden zu werden.

Eine Kupplung der eingangs genannten Art wird allgemein dazu verwendet, zwei Leitungen miteinander zu verbinden. Dazu ist das erste Kupplungsteil mit einer ersten Leitung und das zweite Kupplungsteil mit einer zweiten Leitung verbunden oder verbindbar.

Eine der beiden miteinander zu verbindenden Leitungen kann jedoch auch ein gehäuseseitiger Anschluss eines Gerätes, beispielsweise einer Pumpe, sein, wobei dann das erste Kupplungsteil oder das zweite Kupplungsteil gehäuseseitig befestigt oder befestigbar ist.

Ein Beispiel für die Anwendung der eingangs genannten Kupplung ist das Anschließen eines Spül- und/oder Saugschlauches an eine Spül- und/oder Saugquelle für medizinische Anwendungen. Beispielsweise werden bei chirurgischen Eingriffen, insbesondere in der minimal-invasiven, endoskopgestützten Chirurgie Saug- und/oder Spülinstrumente verwendet, um das Operationsgebiet zu spülen und Flüssigkeiten und/oder Gewebe aus dem Operationsgebiet abzusaugen. Ein solches Saug- und/oder Spülinstrument wird über einen Schlauch mittels der eingangs genannten Kupplung mit der Saug- und/oder Spülquelle verbunden.

Bei einer Verwendung der eingangs genannten Kupplung für ein medizinisches Instrument werden insbesondere hohe Anforderungen an die leichte Handhabbarkeit, an die ausreichende Reinigbarkeit und an eine geringe Baugröße gestellt. Insbesondere ist es erwünscht, dass die beiden Kupplungsteile möglichst ohne Drehbewegung einfach in Längsrichtung der Kupplungsteile zusammensteckbar sind.

Die aus dem oben genannten deutschen Gebrauchsmuster bekannte Kupplung wird ebenfalls auf medizinischem Gebiet verwendet, beispielsweise bei Dialysatoren oder Oxygenatoren für Blut.

Bei der bekannten Kupplung sind die am ersten Kupplungsteil angeordneten ersten Verriegelungsmittel in Form von sechs umfänglich in der Wand des ersten Kupplungsteils verteilten Kugeln ausgebildet, die im wesentlichen quer zur Längsrichtung der Kupplung, das heißt, in radialer Richtung, beweglich sind. Die am zweiten Kupplungsteil ausgebildeten zweiten Verriegelungsmittel sind in Form einer am Außenumfang des zweiten Kupplungsteils vorhandenen Nut ausgebildet, die in Längsrichtung durch zwei Ringflansche begrenzt ist.

Die Arretiermittel sind bei der bekannten Kupplung durch eine am ersten Kupplungsteil angeordnete und diese außen umgebende in Längsrichtung verschiebbare Hülse gebildet. Zum Verbinden des ersten Kupplungsteils mit dem zweiten Kupplungsteil wird das zweite Kupplungsteil in das erste Kupplungsteil eingeführt, bis die Nut auf Höhe der Kugeln zu liegen kommt. Beim Einführen des zweiten Kupplungsteils in das erste Kupplungsteil muss die äußere Hülse des ersten Kupplungsteils von Hand gegen die Kraft einer Federvorspannung in die Entarretierstellung zurückgezogen und in dieser Stellung gehalten werden, damit beim Einführen des zweiten Kupplungsteils die Kugeln in ihre Entriegelungsstellung bewegbar sind. Erst nach vollständigem Einführen des zweiten Kupplungsteils in das erste Kupplungsteil, das heißt, wenn die Kugeln auf Höhe der Nut liegen, kann dann die Hülse losgelassen werden, die sich dann aufgrund ihrer Vorspannung in die Arretierstellung bewegt und die Kugeln mit der Nut in Eingriff hält, wodurch die beiden Kupplungsteile in Längsrichtung zusammengehalten werden.

Zum Lösen der bekannten Kupplung wird in umgekehrter Reihenfolge vorgegangen, das heißt, die Hülse wird zunächst von der Arretierstellung in die Entarretierstellung zurückgezogen, wonach dann das zweite Kupplungsteil von Hand aus dem ersten Kupplungsteil herausgezogen beziehungsweise das erste Kupplungsteil von Hand von dem zweiten Kupplungsteil abgezogen werden kann.

Obwohl die beiden Kupplungsteile der bekannten Kupplung ohne Drehbewegung miteinander verbunden werden können, besteht ein Nachteil der bekannten Kupplung darin, dass insbesondere beim Lösen der Kupplung, wenn das erste Kupplungsteil von dem zweiten Kupplungsteil abgezogen wird, mit erhöhter Handkraft gearbeitet werden muss, um die Kugeln mit der Nut außer Eingriff zu bringen, wobei insbesondere dann, wenn die Hülse beim Abziehen des ersten Kupplungsteils vom zweiten Kupplungsteil nicht streng in ihrer Entarretierstellung festgehalten wird, es zu einem Verhaken der Kupplungsteile beim Trennen der Kupplung kommen kann.

Ferner ist die in dieser Kupplung vorhandene Dichtung so angeordnet, dass diese nur schwer zugänglich ist.

Der Erfindung liegt daher die Aufgabe zugrunde, eine Kupplung der eingangs genannten Art dahingehend weiterzubilden, dass die Leichtgängigkeit der Kupplung insbesondere beim Trennen verbessert ist.

Ferner soll die Zugänglichkeit der Dichtung in einer solchen Kupplung verbessert werden.

Ferner soll das erste Kupplungsteil auf einfachere Weise hergestellt werden.

Erfindungsgemäß wird diese Aufgabe hinsichtlich der eingangs genannten Kupplung dadurch gelöst, dass im ersten Kupplungsteil ein in dessen Längsrichtung beweglicher Schieber angeordnet ist, der beim Einführen des zweiten Kupplungsteils in das erste Kupplungsteil von einer vorgeschobenen in eine zurückgezogene Stellung bewegt wird, dass der Schieber in die vorgeschobene Stellung vorgespannt ist, und dass an einem dem ersten Kupplungsteil zugewandten Ende des zweiten Kupplungsteils Dichtungsmittel angeordnet sind, die in einem Zustand, in dem das erste Kupplungsteil mit dem zweiten Kupplungsteil verbunden ist, einen Innenraum der Kupplung gegenüber einem Fluid abdichten.

Die Verwendung des Begriffs dem ersten bzw. zweiten Kupplungsteil zugewandt bzw. abgewandt, bezieht sich jeweils auf die Position der Kupplungsteile in verbundenem Zustand.

Erfindungsgemäß wird die Aufgabe ferner durch ein Verfahren zum Herstellen eines ersten Kupplungsteils einer Kupplung für ein medizinisches Instrument zum Verbinden zweier Leitungen mit den folgenden Schritten gelöst, nämlich

Bereitstellen eines zweiten Teils eines Körpers des ersten Kupplungsteils, wobei das zweite Teil röhrenförmig ausgebildet ist, und wobei dieses an seiner inneren Mantelfläche zumindest einen ersten Vorsprung, und an einem dem zweiten Kupplungsteil zugewandten Ende an der äußeren Mantelfläche zumindest einen zweiten Vorsprung aufweist.

Einführen einer inneren Hülse in das zweite Teil, wobei zwischen einem ersten Abschnitt der Hülse und dem ersten Vorsprung des zweiten Teils elastische Elemente vorgesehen sind, die auf die Hülse eine Kraft entgegen der Richtung des Einführens ausüben,

Anbringen einer Sicherung an der inneren Hülse wobei die Sicherung in Richtung des Einführen stromabwärts des ersten Vorsprungs des zweiten Teils an der Hülse angebracht wird um ein Herausfallen der Hülse aus dem zweiten Teil zu verhindern,

Bereitstellen von Verriegelungsmitteln in dem zweiten Teil,

Aufschieben einer äußeren Hülse auf das zweite Teil, wobei die äußere Hülse einen ersten Bereich mit einem kleineren Innendurchmesser und einen zweiten Bereich mit einem größeren Innendurchmesser aufweist.

Verbinden eines ersten Teils des Körpers mit dem zweiten Teil, wobei das erste Teil die Bewegung der äußeren Hülse in einer Richtung entgegen der Richtung ihres Aufschiebens begrenzt und wobei in dem Bereich des zweiten Abschnitts zwischen der äußeren Hülse und dem ersten Teil elastische Elemente vorgesehen sind, die auf die äußere Hülse ein Kraft in Richtung des Aufschiebens ausüben.

Der bei der erfindungsgemäßen Kupplung vorgesehene Schieber, gegen den das zweite Kupplungsteil beim Einführen in das erste Kupplungsteil in Anlage kommt, und der beim weiteren Einführen des zweiten Kupplungsteils gegen die Vorspannung in eine zurückgezogene Stellung bewegt wird, wirkt in der Art eines Ausstoßers unterstützend auf das Trennen des zweiten Kupplungsteils vom ersten Kupplungsteil, wodurch sich die Kupplung mit geringerer Handkraft lösen lässt. Sobald die Arretiermittel zum Lösen der Kupplung in die Entarretierstellung bewegt wurden, übt der in die vorgeschobene Stellung vorgespannte Schieber unmittelbar auf das zweite Kupplungsteil eine in Trennrichtung wirkende Kraft aus, die das Abziehen des ersten Kupplungsteils vom zweiten Kupplungsteil bzw. das Herausziehen des zweiten Kupplungsteils aus dem ersten Kupplungsteil entsprechend der Vorspannung des Schiebers unterstützt. Des weiteren eröffnet der erfindungsgemäß vorgesehene Schieber die Möglichkeit, die nachfolgend noch zu beschreibenden vorteilhaften Funktionen in bevorzugten Weiterbildungen zu realisieren.

Durch das Anordnen der Dichtungsmittel an einem dem ersten Kupplungsteil zugewandten Ende des zweiten Kupplungsteils werden diese ferner für einen Anwender auf einfache Weise zugänglich, zum Beispiel um diese bei Verschleißerscheinungen auszutauschen. Ferner ist der Innenraum der Kupplung gegenüber einer Kontamination durch Fluid geschützt. Ferner ist auch ein in den Leitungen vorhandenes Fluid vor gegebenenfalls in der Kupplung vorhandenen Verschmutzungen effektiv geschützt.

Das erfindungsgemäße Herstellungsverfahren für das erste Kupplungsteil zeichnet sich zum einen durch seine besondere Einfachheit aus, da die meisten Bauteile durch einfaches Aufeinanderschieben bzw. gegebenenfalls zum Beispiel Verschrauben oder Verpressen miteinander verbunden werden können und führt zum anderen zu einer Kupplung mit einer besonders geringen Baugröße, was ebenfalls vorteilhaft ist.

In einer ersten bevorzugten Ausgestaltung hält der Schieber in der vorgeschobenen Stellung die ersten Verriegelungsmittel in der Entriegelungsstellung, wobei letztere dabei die Arretiermittel in der Entarretierstellung halten.

Diese Maßnahme hat einen besonderen Vorteil beim Verbinden und Verriegeln der beiden Kupplungsteile aneinander. Vor dem Einführen des zweiten Kupplungsteils in das erste Kupplungsteil bewirkt der Schieber, indem er die ersten Verriegelungsmittel in der Entriegelungsstellung hält und diese dabei die Arretiermittel in der Entarretierstellung halten, dass die Arretiermittel zum Verbinden der beiden Kupplungsteile nicht zuvor vom Benutzer in die Entarretierstellung bewegt und dort gehalten werden müssen, wie dies bei der bekannten Kupplung der Fall ist, bei der die Hülse vor dem Verbinden der beiden Kupplungsteile zurückgezogen und in dieser zurückgezogenen Stellung von Hand gehalten werden muss. In der erfindungsgemäßen Kupplung muss gemäß der zuvor genannten Ausgestaltung lediglich das zweite Kupplungsteil in das erste Kupplungsteil eingeführt beziehungsweise das erste Kupplungsteil auf das zweite Kupplungsteil aufgesteckt werden, ohne dass die Arretiermittel betätigt werden müssen. Der weitere Vorteil dieser Maßnahme besteht darin, dass die Verriegelungsmittel, insbesondere wenn diese wie bei der bekannten Kupplung als Kugeln ausgebildet sind, bei getrennten Kupplungsteilen unverlierbar in ihren Ausnehmungen in der Wand des ersten Kupplungsteils gehalten werden können. Bei der bekannten Kupplung wird dies dadurch realisiert, dass die Taschen, in denen die Kugeln aufgenommen sind, radial nach innen schmaler werden, was jedoch den Nachteil hat, dass die Eindringtiefe der Kugeln in die Nut gering ist und dadurch im verbundenen Zustand der beiden Kupplungsteile die axiale Zusammenhaltekraft der Kupplung begrenzt ist. Bei der vorliegenden Ausgestaltung können demnach Verriegelungsmittel in Form von Kugeln in ihren Aufnahmen in dem ersten Kupplungsteil frei beweglich sein, das heißt, die Aufnahmen müssen nicht radial nach innen verjüngt ausgestaltet sein, wodurch eine größere Eingriffstiefe der Kugeln in einer entsprechenden tieferen Nut und eine einfachere Herstellbarkeit der Aufnahmen, die als zylindrische Bohrungen ausgeführt werden können, ermöglicht wird. Zur größeren Sicherheit kann jedoch auch eine Verjüngung vorzugsweise in Form einer Stufenbohrung vorliegen. Eine Stufenbohrung hat den Vorteil, dass sie konstruktiv einfach ist und dennoch zu einer angemessenen Eindringtiefe führt.

In einer weiteren bevorzugten Ausgestaltung gibt der Schieber in der zurückgezogenen Stellung die ersten Verriegelungsmittel frei, damit diese mit den zweiten Verriegelungsmitteln in Eingriff kommen können, wonach die Arretiermittel von der Entarretierstellung in die Arretierstellung bewegbar sind.

Auch diese Maßnahme trägt vorteilhafterweise zu einer weiter verbesserten Steuerung der Bewegung der Verriegelungsmittel zwischen der Verriegelungsstellung und der Entriegelungsstellung bei, wodurch die Handhabung und insbesondere die Leichtgängigkeit der Kupplung auch beim Verbinden der beiden Kupplungsteile verbessert wird, insbesondere wenn die Arretiermittel, wie in einer weiteren bevorzugten Ausgestaltung vorgesehen ist, in die Arretierstellung vorgespannt sind.

In einer weiteren bevorzugten Ausgestaltung ist die Vorspannung des Schiebers ausreichend bemessen, um das zweite Kupplungsteil aus dem ersten Kupplungsteil auszustoßen, wenn die Arretiermittel von der Arretierstellung in die Entarretierstellung bewegt werden.

Durch diese Ausgestaltung wirkt der erfindungsgemäß vorgesehene Schieber im ersten Kupplungsteil vorteilhafterweise als Ausstoßer, wodurch das Trennen der beiden Kupplungsteile mit sehr geringer Handkraft ermöglicht wird. Zum Trennen der beiden Kupplungsteile müssen lediglich die Arretiermittel von der Arretierstellung in die Entarretierstellung bewegt werden, wodurch die Verriegelungsmittel von der Verriegelungsstellung in die Entriegelungsstellung bewegbar sind, so dass der Schieber dann das zweite Kupplungsteil aus dem ersten Kupplungsteil herausdrückt.

In einer weiteren bevorzugten Ausgestaltung bewegen die Arretiermittel bei der Bewegung von der Entarretierstellung in die Arretierstellung die ersten Verriegelungsmittel aus der Entriegelungsstellung in die Verriegelungsstellung.

Hierbei ist von Vorteil, dass die Arretiermittel die Verriegelungsmittel nicht nur in Eingriff halten beziehungsweise in der Entarretierstellung freigeben, sondern auch die Bewegung der Verriegelungsmittel aktiv steuern, wodurch die Betriebssicherheit der erfindungsgemäßen Kupplung weiter verbessert wird.

In diesem Zusammenhang ist es bevorzugt, wenn die ersten Verriegelungsmittel zumindest einen Formkörper, vorzugsweise zumindest eine Kugel, aufweisen, der in einer Wand des ersten Kupplungsteils angeordnet ist, und die Arretiermittel eine die Wand des ersten Kupplungsteils umgebende Hülse aufweisen, die einen ersten Abschnitt größeren Innendurchmessers und einen zweiten Abschnitt kleineren Innendurchmessers aufweist, wobei zwischen dem ersten und dem zweiten Abschnitt eine Anlaufschräge vorhanden ist.

Diese Maßnahme stellt eine vorteilhafterweise konstruktiv einfache Ausgestaltung dar, um die ersten Verriegelungsmittel bei einer Bewegung der Arretiermittel von der Entarretierstellung in die Arretierstellung aus der Entriegelungsstellung in die Verriegelungsstellung zu bewegen. Die Anlaufschräge sorgt dabei für eine vorteilhafterweise besonders hohe Leichtgängigkeit des Verriegelungsmechanismus.

In einer weiteren bevorzugten Ausgestaltung sind die Arretiermittel durch eine in Längsrichtung erfolgende Bewegung von der Arretier- in die Entarretierstellung bewegbar.

Diese Maßnahme hat den Vorteil, dass lediglich in Längsrichtung der Kupplung erfolgende Bewegungen zum Trennen und Lösen der Kupplung erforderlich sind, was der Richtung der Verbindung und der Trennung der beiden Kupplungsteile entspricht.

Gemäß der zuvor genannten Ausgestaltung sind der erste und der zweite Abschnitt der Hülse dann vorteilhafterweise in Längsrichtung hintereinander angeordnet und die Anlaufschräge erstreckt sich in Längsrichtung.

Alternativ hierzu kann es jedoch ebenso zweckmäßig und bevorzugt sein, wenn die Arretiermittel durch eine in Umfangsrichtung erfolgende Bewegung von der Arretierin die Entarretierstellung bewegbar sind.

Diese Maßnahme hat den Vorteil, dass die Kupplung in Längsrichtung kurzbauend ausgeführt werden kann, weil kein Bauraum für einen axialen Bewegungsweg der Arretiermittel benötigt wird. Eine in Umfangsrichtung erfolgende Bewegung der Arretiermittel bedeutet jedoch nicht, dass die beiden Kupplungsteile durch eine Drehung miteinander verbunden werden müssen. Vielmehr ist die erfindungsgemäße Kupplung auch bei dieser Ausgestaltung als Steckkupplung ausgebildet.

Gemäß der zuvor genannten Ausgestaltung ist in einer konstruktiv besonders einfachen Ausgestaltung entsprechend vorgesehen, dass der erste und zweite Abschnitt in Umfangsrichtung hintereinander angeordnet sind und sich die Anlaufschräge in Umfangsrichtung erstreckt.

In einer weiteren bevorzugten Ausgestaltung ist der Schieber in Form einer innerhalb des ersten Kupplungsstücks angeordneten Hülse ausgebildet, die an einer Innenseite des ersten Kupplungsteils geführt ist, wobei ein Anschlag für die Hülse in der vorgeschobenen Stellung vorgesehen ist.

Diese Maßnahme stellt eine konstruktiv besonders einfache Möglichkeit dar, den Schieber für die zuvor genannten Funktionen auszubilden, wobei der Schieber durch die Ausgestaltung als Hülse einen Fluidstrom durch die Kupplung hindurch in keiner Weise beeinträchtigt.

In einer weiteren bevorzugten Ausgestaltung ist ein vorderes Ende des Schiebers mit einer Anlaufschräge versehen.

Diese Maßnahme hat den Vorteil, dass der Schieber bei seiner Bewegung von der zurückgezogenen in die vorgeschobene Stellung auf die Verriegelungsmittel auflaufen kann und somit ein Blockieren der Bewegung des Schiebers in die vorgeschobene Stellung vermieden wird, sofern der Schieber dazu vorgesehen ist, wie in einer der zuvor genannten Ausgestaltungen beschrieben wurde, die ersten Verriegelungsmittel in der Entriegelungsstellung zu halten. Die Anlaufschräge kann gerade, konvex oder konkav ausgebildet sein.

In einer weiteren bevorzugten Ausgestaltung weisen die Dichtungsmittel einen O-Ring auf, der in einer Ringnut in dem dem ersten Kupplungsteil zugewandten Ende des zweiten Kupplungsteils angeordnet ist und der in einem Zustand, in dem das erste Kupplungsteil mit dem zweiten Kupplungsteil verbunden ist, gegen die Innenseite einer Bohrung in dem ersten Kupplungsteil anliegt.

Diese Ausgestaltung stellt konstruktiv eine besonders einfache Ausführung der Dichtungsmittel dar, die auch gleichzeitig preiswert herzustellen sind. Ein so angebrachter O-Ring lässt sich auf einfache Weise durch Aufschneiden oder Herausrollen aus der Nut entfernen und zum Beispiel durch einen neuen O-Ring ersetzen, was das Austauschen der Dichtungsmittel deutlich vereinfacht.

In einer Ausgestaltung des vorgenannten Verfahrens weisen die elastischen Mittel zumindest eine Druckfeder auf.

Druckfedern behalten über lange Zeit ihre Elastizität bei, wodurch auch auf lange Sicht die Ausübung der von den elastischen Elementen ausgeübten Kraft sichergestellt werden kann.

In einer weiteren Ausgestaltung des oben genannten Verfahrens weisen die Verriegelungsmittel zumindest eine Kugel auf, die in einer Richtung quer zur Längsachse des ersten Kupplungsteils verschiebbar ist.

Diese Maßnahme hat den Vorteil, dass zum einen die Verwendung von Kugeln konstruktiv einfach ist, da diese lediglich in eine Querbohrung eingeführt werden müssen und zum anderen sorgen Kugeln durch ihre runde Form für eine besondere Leichtgängigkeit eines nach dem Verfahren erhaltenen Kupplungsteils.

In einer weiteren Ausgestaltung der zuvor genannten Maßnahme ist der erste Vorsprung eine Ringwulst und/oder der zweite Vorsprung ist ein Ringflansch.

Eine Ringwulst oder ein Ringflansch sind vorteilhaft, dass diese zum einen leicht während des Herstellungsprozesses des zweiten Teils gebildet werden können und zum zweiten dadurch, dass diese sich um den gesamten inneren bzw. äußeren Durchmesser des zweiten Teils erstrecken. Daher gibt es während der Herstellung des Kupplungsteils keine Probleme der Ausrichtung der elastischen Elemente mit dem zweiten Teil, so dass das Herstellungsverfahren deutlich einfacher gemacht wird.

In einer weiteren Ausgestaltung des oben genannten Verfahrens ist die Sicherung eine Mutter, die auf die innere Hülse aufgeschraubt wird.

Die Verwendung einer Mutter als Sicherung hat den Vorteil, dass diese einfach mit dem zweiten Teil verbunden werden kann, was die Herstellung deutlich vereinfacht.

Ferner kann es sich bei der Verwendung einer Mutter um eine lösbare Verbindung handeln, so dass hierdurch ein Kupplungsteil erhalten wird, das gegebenenfalls zum Beispiel zu Wartungszwecken auch auseinandergebaut werden kann. Außerdem kann bei der Verwendung einer solchen Mutter diese auch dazu verwendet werden, die Vorspannung der inneren Hülse einzustellen.

In einer weiteren Ausgestaltung des oben genannten Verfahrens ist die Sicherung einstückig mit der inneren Hülse ausgebildet.

Bei dieser Ausgestaltung, die alternativ zu der vorherigen zur Anwendung kommen kann, bilden die innere Hülse und die Sicherung ein Bauteil. Hierbei kann die Sicherung z.B. die Form um an der Hülse angeordneten Rastfingern annehmen, die z.B. hinter eine an der inneren Mantelfläche des zweiten Teils angeordnete Ringwulst greifen. Bei dieser Ausgestaltung bilden das Einführen der Hülse und das Anbringen der Sicherung zwei Abschnitte eines Arbeitsvorgangs, wobei das Anbringen der Sicherung z.B. in dem Einführen der Hülse bis zum Einrasten der zuvor beschriebenen Rastfinger bestehen kann. Dies hat den Vorteil, dass dadurch das Verfahren noch effizienter gemacht wird. Ferner hat dies den Vorteil, dass die Hülse und die Sicherung preisgünstig z.B. als Spritzgussteil hergestellt werden können.

Die erfindungsgemäße Kupplung ist vorzugsweise aus einem biokompatiblen Material gefertigt und dampfsterilisierbar, außerdem vorzugsweise Gas- und Plasmasterilisierbar und chemisch und thermisch reinig- und desinfizierbar.

Weitere Vorteile und Merkmale ergeben sich aus der nachfolgenden Beschreibung und den beigefügten Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung wird hier nachstehend in Bezug auf die beiliegenden Zeichnungen näher beschrieben, in welchen:
- Fig. 1: eine erfindungsgemäße Kupplung im Längsschnitt im getrennten Zustand der beiden Kupplungsteile zeigt; und
- Fig. 2: die Kupplung von Fig. 1 im verbundenen Zustand im Längsschnitt zeigt.

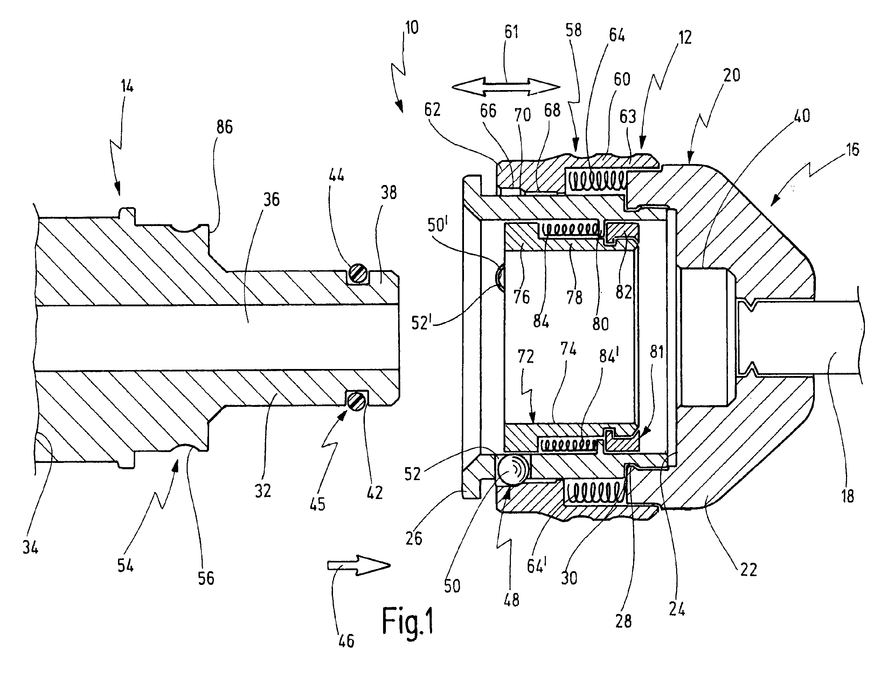

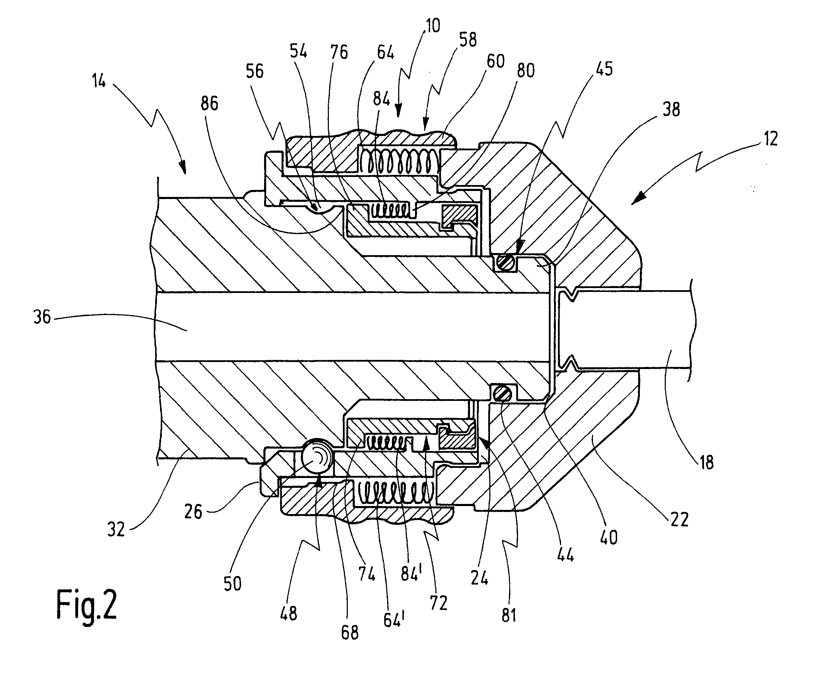

In den Fig. 1 und 2 ist eine Kupplung für ein medizinisches Instrument in ihrer Gesamtheit mit der Bezugsziffer 10 bezeichnet.

Die Kupplung 10 weist ein erstes Kupplungsteil 12 und ein zweites Kupplungsteil 14 auf.

In dem dargestellten Beispiel ist das erste Kupplungsteil 12 an einem hinteren Ende 16 lösbar mit einem Schlauch 18 verbunden. Hierbei ist ein Ende des Schlauchs 18 in das hintere Ende 16 des ersten Kupplungsteils 12 eingeschraubt.

Durch die Kupplung 10 wird im geschlossenen Zustand eine Flüssigkeit oder ein Gas geleitet, oder ein medizinisches Instrument eingeführt.

Das erste Kupplungsteil 12 weist einen Körper 20 auf, der hier aus einem ersten Teil 22 und einem zweiten Teil 26 besteht. Das erste Teil 22 weist an einem dem zweiten Kupplungsteil 14 zugewandten Ende eine Stufenbohrung 24 auf. An dem dem zweiten Kupplungsteil 14 zugewandten Ende der Stufenbohrung 24 weist das erste Teil 22 ein in die Stufenbohrung 24 hineinstehendes Innengewinde 28 auf. Dieses Innengewinde 28 greift in ein entsprechendes Außengewinde 30 ein, das an dem dem zweiten Kupplungsteil 14 abgewandten Ende des zweiten Teils 26 des Körpers 20 in der äußeren Mantelfläche des zweiten Teils 26 angeordnet ist. Auf diese Weise sind das erste Teil 22 und das zweite Teil 26 fest miteinander verschraubt.

Das zweite Kupplungsteil 14 weist einen Körper 32 auf, der im vorliegenden Fall einstückig als Drehteil ausgebildet ist. An einem dem ersten Kupplungsteil 12 abgewandten Ende 34 des Körpers 32 ist das zweite Kupplungsteil 14 im vorliegenden Fall fest mit einem hier nicht dargestellten medizinischen Instrument verbunden. Eine Durchgangsöffnung 36, z.B. zur Durchleitung von Fluid erstreckt sich durch den Körper 32.

An einem dem ersten Kupplungsteil 12 zugewandten Ende 38 des Körpers 32 ist in der äußeren Mantelfläche des Körpers 32 eine Ringnut 42 eingebracht. In dieser Ringnut liegt ein O-Ring 44 aus einem elastischen Material, zum Beispiel einem Kautschukmaterial, der hier die Dichtungsmittel 45 des zweiten Kupplungsteils 14 bildet. Die Größe des O-Rings 44 ist hierbei so ausgewählt, dass dieser fest in der Ringnut 42 zu liegen kommt. Der Außendurchmesser des Endes 38 des Körpers 32 entspricht hierbei etwa dem lichten Innendurchmesser eines zweiten Abschnitts 40 der Stufenbohrung 24 in dem ersten Teil 22 des Körpers 20 des ersten Kupplungsteils 12.

Das zweite Kupplungsteil 14 ist gemäß einem Pfeil 46, d.h. in Längsrichtung der Kupplungsteile 12 und 14, in das erste Kupplungsteil 12 einführbar, um das erste Kupplungsteil 12 mit dem zweiten Kupplungsteil 14 zu verbinden. Hierbei ist in Fig. 1 der getrennte Zustand der Kupplungsteile 12 und 14 dargestellt, während in Fig. 2 der verbundene Zustand der beiden Kupplungsteile 12 und 14 dargestellt ist.

An dem ersten Kupplungsteil 12 sind erste Verriegelungsmittel 48 angeordnet. Die ersten Verriegelungsmittel 48 weisen im vorliegenden Ausführungsbeispiel drei Kugeln 50, 50' auf. Die drei Kugeln 50, 50' sind in Umfangsrichtung zueinander um 120° versetzt angeordnet, so dass in Fig. 1 nur zwei und in Fig. 2 nur eine der drei Kugeln 50, 50' zu sehen sind.

Anstelle von Kugeln können die ersten Verriegelungsmittel 48 auch andere Formkörper aufweisen, beispielsweise Stifte oder dergleichen, wobei jedoch die Kugeln 50, 50' als erste Verriegelungsmittel 48 bevorzugt sind.

Die Kugeln 50, 50' sind jeweils einzeln in Taschen 52, 52' aufgenommen, die in dem zweiten Teil 26 des Körpers 20 des ersten Kupplungsteils 12 in Form von quer zur Längsrichtung radial durchgehenden Bohrungen in der Wand des zweiten Teils 26 ausgebildet sind. Entsprechend der Anzahl von drei Kugeln 50, 50' sind drei solche Taschen 52, 52' in dem ersten Kupplungsteil 12 vorhanden.

Anstatt der hier dargestellten durchgehenden Bohrungen können die Taschen auch in Form von Stufenbohrungen ausgebildet sein, die sich in Richtung der Innenseite des zweiten Teils 26 verjüngen und somit ein Herausfallen der Kugeln 50, 50' verhindern.

In den Taschen 52, 52' sind die Kugeln 50, 50' quer zur Längsrichtung frei beweglich.

Am zweiten Kupplungsteil 14 sind entsprechend zweite Verriegelungsmittel 54 angeordnet. Im vorliegenden Ausführungsbeispiel weisen die zweiten Verriegelungsmittel 54 eine Ringnut 56 auf. Die Ringnut 56 ist vollumfänglich in der äußeren Mantelfläche des Körpers 32 des zweiten Kupplungsteils 14 ausgebildet. Aufgrund der vollumfänglichen Ausgestaltung der Ringnut 56 kann das zweite Kupplungsteil 14 unabhängig von der Drehorientierung des zweiten Kupplungsteils 14 relativ zum ersten Kupplungsteil 12 mit dem ersten Kupplungsteil 12 verbunden werden.

Die Kugeln 50, 50' sind zwischen einer in Fig. 1 dargestellten Entriegelungsstellung, in der sie sich in einer radial äußeren Position in den Taschen 52, 52' befinden und einer Verriegelungsstellung beweglich, in der sie radial nach innen aus den Taschen 52, 52' heraus hervorstehen und in die Nut 56 eingreifen. Die Nut 56 weist entsprechend der Ausgestaltung der ersten Verriegelungsmittel 48 als Kugeln 50, 50' eine konkave Form auf.

Am ersten Kupplungsteil 12 sind ferner Arretiermittel 58 angeordnet, die eine Hülse 60 aufweisen, die zum größten Teil um das zweite Teil 26 des Körpers 20 des ersten Kupplungsteils 12 herum angeordnet ist. Die Arretierungsmittel 58, d.h. im vorliegenden Ausführungsbeispiel die Hülse 60, sind zwischen einer in Fig. 1 dargestellten Entarretierstellung, in der die Hülse 60 in Richtung des hinteren Endes 16 des ersten Kupplungsteils 12 zurückgezogen ist und einer in Fig. 2 dargestellten Arretierstellung, in der die Hülse von dem hinteren Ende 16 des ersten Kupplungsteils 12 nach vorne geschoben ist, gemäß dem Doppelpfeil 61 beweglich.

Die Hülse 60 weist hierbei einen ersten Bereich 62 mit einem kleineren Innendurchmesser und einen zweiten Bereich 63 mit einem größeren Innendurchmesser auf. In dem Raum, der zwischen der inneren Mantelfläche des zweiten Bereichs 63 und der äußeren Mantelfläche des zweiten Teils 26 gebildet wird, sind hierbei Druckfedern angeordnet, die beispielhaft mit dem Bezugszeichen 64, 64' bezeichnet sind. Im vorliegenden Beispiel sind mehrere Druckfedern 64, 64' dargestellt, es ist aber auch möglich, eine einzelne Druckfeder zu verwenden, die sich zum Beispiel helixförmig um die äußeren Mantelfläche des zweiten Teils 26 erstreckt.

Die Druckfedern 64, 64' stützen sich an einem Ende gegen das erste Teil 22 des ersten Kupplungsteils 12 und am anderen Ende gegen die Hülse 60 ab, so dass die Hülse in die in Fig. 2 dargestellte vorgeschobene Position vorgespannt ist. In Fig. 1, in der die Hülse 60 in die Entarretierstellung zurückgezogen ist, sind die Federn 64, 64' entsprechend gespannt.

An ihrem dem zweiten Kupplungsteil 14 zugewandten Ende weist die Hülse 60 einen ersten Abschnitt 66 größeren Innendurchmessers und einen sich daran in Längsrichtung zum hinteren Ende 16 hin anschließenden zweiten Abschnitt 68 kleineren Innendurchmessers auf. Der erste Abschnitt 66 und der zweite Abschnitt 68 sind durch eine Anlaufschräge 70 miteinander verbunden, die sich in Längsrichtung der Kupplung 10 erstreckt.

Innerhalb des ersten Kupplungsteils 12, genauer gesagt innerhalb des zweiten Teils 26 ist ein Schieber 72 angeordnet. Der Schieber 72 ist in Form einer Hülse 74 ausgestaltet. Diese Hülse 74 weist einen ersten Abschnitt 76 auf, der einen Außendurchmesser besitzt, der in etwa dem Innendurchmesser des zweiten Teils 26 in diesem Bereich entspricht, so dass die Hülse 74 über den ersten Abschnitt 76 an der Innenseite des ersten Kupplungsteils 12 geführt ist.

Der Schieber 72 ist zwischen einer in Fig. 1 dargestellten vorgeschobenen Stellung und einer in Fig. 2 dargestellten zurückgezogenen Stellung in Längsrichtung gemäß dem Doppelpfeil 61 beweglich. Um den Bewegungsweg des Schiebers 72 in Form der Hülse 74 in Längsrichtung in Richtung des Pfeils 46 zu begrenzen, weist das zweite Teil 26 an seiner Innenseite eine Ringwulst 80 auf, deren Innendurchmesser in etwa dem Außendurchmesser eines zweiten Abschnitts 78 der Hülse 74 entspricht und der kleiner ist als der Außendurchmesser des ersten Abschnitts 76 der Hülse 74. Durch diese Ausführung wird die Führung der Hülse 74 in dem ersten Kupplungsteil 12 noch weiter verbessert.

Um ein Herausfallen der Hülse aus dem zweiten Teil 26 zu verhindern, ist ferner an dem dem zweiten Kupplungsteil 14 abgewandten Ende des Schiebers 72 eine Sicherung 81 in Form einer Mutter 82 angebracht, deren Außendurchmesser in etwa dem Innendurchmesser des zweiten Teils 26 entspricht und der größer ist als der Innendurchmesser der Ringwulst 80. Hierdurch wird ein Herausziehen oder Herausfallen der Hülse 74 aus dem zweiten Teil 26 verhindert.

Der Schieber 72 ist ferner durch Druckfedern, die hier beispielhaft mit den Bezugsziffern 84, 84' bezeichnet sind und die sich auf der einen Seite gegen die Ringwulst 80 und auf der anderen Seite gegen den ersten Abschnitt 76 der Hülse 74 abstützen, in der in Fig. 1 dargestellten Position vorgespannt. In der Stellung in Fig. 2 sind die Druckfedern 84, 84' entsprechend vorgespannt. Es ist auch hier klar, dass anstatt mehrerer Druckfedern 84, 84' nur eine einzige Druckfeder verwendet werden kann, die sich zum Beispiel helixweise um die Hülse 74 erstreckt.

In dem in Fig. 1 dargestellten Zustand ist das zweite Kupplungsteil 14 von dem ersten Kupplungsteil 12 getrennt. In diesem Zustand befindet sich der Schieber 72 in seiner vorgeschobenen Stellung, in der der Schieber 72 die ersten Verriegelungsmittel 48 in ihrer Entriegelungsstellung hält, indem der erste Abschnitt 76 der Hülse 74 des Schiebers 72 die Kugeln 50, 50' in den Taschen 52, 52' radial nach außen drängt. Da die Kugeln 50, 50' in den Taschen 52, 52' durch den Schieber 72 in Fig. 1 radial nach außen gehalten werden, sind auch die Arretiermittel 58, d.h. im vorliegenden Ausführungsbeispiel die Hülse 60 in ihrer Entarretierungsstellung festgehalten. Die Kugeln 50, 50' verhindern in ihrer radial äußeren festgehaltenen Stellung, dass die Hülse 60 mit ihrer Anlaufschräge 70 an den Kugeln 50, 50' vorbeibewegt werden kann.

Wird nun das zweite Kupplungsteil 14 in das erste Kupplungsteil 12 eingeführt, kommt die Stirnseite eines Abschnitts 86 des Körpers 32 des zweiten Kupplungsteils 14 mit der Stirnseite des ersten Abschnitts 76 der Hülse 74 in Anlage und beim weiteren Einführen des zweiten Kupplungsteils 14 in Richtung des Pfeils 46 wird der Schieber 72 in die zurückgezogene Stellung gemäß Fig. 2 bewegt. In dieser Stellung gibt der Schieber 72 die ersten Verriegelungsmittel 48, hier die Kugeln 50, 50', frei, die dann sobald die zweiten Verriegelungsmittel 54, hier die Nut 56, auf Höhe der ersten Verriegelungsmittel 48 zu liegen kommen, mit den zweiten Verriegelungsmitteln 54 in Eingriff kommen können. Dieses in Eingriff Kommen der ersten Verriegelungsmittel 48 mit den zweiten Verriegelungsmitteln 54 erfolgt in der Art eines Einrastens, indem die Arretierungsmittel 58 in Form der Hülse 60 die Kugeln 50, 50' mittels der Anlaufschräge 70 aktiv in die Nut 56 drücken. Die Arretierungsmittel 58 bewegen sich dabei aufgrund der Vorspannung der Hülse 60 selbsttätig in die Arretierstellung.

Aus der vorhergehenden Beschreibung wird deutlich, dass zum Verbinden des ersten Kupplungsteils 12 mit dem zweiten Kupplungsteil 14 lediglich das erste Kupplungsteil 12 auf das zweite Kupplungsteil 14 aufgesteckt werden muss, bzw. das zweite Kupplungsteil 14 in das erste Kupplungsteil 12 eingeführt werden muss, wobei die Verriegelung und Arretierung der Kupplung 10 dann aufgrund des Schiebers 72 vollständig automatisch in einer Art Verrastung erfolgt. Im Unterschied zum Stand der Technik müssen die Arretierungsmittel 58 beim Verschieben der beiden Kupplungsteile nicht von Hand in die Entarretierstellung bewegt und in dieser festgehalten werden, da dies von dem Schieber 72 in seiner vorgeschobenen Stellung bewerkstelligt wird.

Im verbundenen Zustand liegt nun der O-Ring 44 in dem zweiten Abschnitt 40 der Stufenbohrung 24 an der Innenwand an, wodurch der gesamte Innenraum der Kupplung 10 gegenüber einem Eindringen von Fluid, das gegebenenfalls durch den Schlauch 18 sowie die Durchgangsöffnung 36 fließt, abgedichtet wird.

Zum Trennen der Kupplung 10 wird ausgehend von dem in Fig. 2 dargestellten verbunden Zustand wie folgt vorgegangen.

Die Arretierungsmittel 58, hier die Hülse 60, werden aus der in Fig. 2 dargestellten vorgeschobenen Arretierstellung in die in Fig. 1 dargestellte zurückgezogene Entarretierstellung gegen die Vorspannung zurückbewegt. Da dann der erste Abschnitt 66 der Hülse 60 auf Höhe der Kugeln 50, 50' zu liegen kommt, besitzen diese nun eine radiale Beweglichkeit nach außen. Aufgrund der Vorspannung des Schiebers 72 in seine in Fig. 1 dargestellte vorgeschobene Stellung drückt dieser nun gegen das zweite Kupplungsteil 14 und stößt dieses je nach Einstellung der Vorspannung des Schiebers 72 aus oder unterstützt zumindest eine vom Benutzer ausgeübte Zugkraft, wodurch das Trennen der Kupplung 10 besonders einfach zu handhaben ist. Sobald die Kugeln 50, 50' mit der Nut außer Eingriff gebracht sind, d.h. sich in ihrer Entriegelungsstellung befinden, werden sie dort auch gehalten, und zwar zunächst durch das zweite Kupplungsteil 14. Wenn dieses entgegen der Richtung des Pfeils 46 in Fig. 1 an den Kugeln 50, 50' vorbeibewegt wurde, übernimmt der erste Abschnitt 76 der Hülse 74 des Schiebers 72 das Halten der Kugeln 50, 50' in ihrer Entriegelungsstellung, so dass die Hülse 60 nun bereits losgelassen werden kann. Der Schieber 72 bewegt sich selbsttätig in die in Fig. 1 dargestellte vorgeschobene Stellung, wonach wieder der Ausgangszustand gemäß Fig. 1 erreicht ist, in dem die Arretierungsmittel 58 in ihrer Entarretierstellung und ersten Verriegelungsmittel 48 in ihrer Entriegelungsstellung gehalten sind.

Während die Arretiermittel 58 in dem gezeigten Ausführungsbeispiel von der Arretierstellung in die Entarretierstellung bzw. umgekehrt in Längsrichtung der Kupplung beweglich sind, kann auch vorgesehen sein, dass diese Bewegung als Umfangsbewegung bzw. Drehbewegung der Arretiermittel 58 relativ zu dem Körper 20 des ersten Kupplungsteils 12 erfolgt. Entsprechend wären dann der erste Abschnitt 66 größeren Innendurchmessers und der zweite Abschnitt 68 kleineren Innendurchmessers nicht in Längsrichtung hintereinander sondern in Umfangsrichtung hintereinander angeordnet, wobei sich dann die Anlaufschräge 70 in Umfangsrichtung erstrecken würde.

Das erste Kupplungsteil 12 kann auf folgende Weise hergestellt werden. Hierzu wird zuerst das zweite Teil 26 des Körpers 20 des ersten Kupplungsteils 12 bereitgestellt. In dieses zweite Teil 26 wird durch axiales Einschieben in Richtung des Pfeils 46 die Hülse 74 eingeschoben, wobei die Druckfedern 84, 84' in den zwischen der äußeren Mantelfläche des zweiten Abschnitts 78 und der inneren Mantelfläche des zweiten Teils 26 gebildeten Raums eingebracht werden. Im nächsten Schritt wird auf den hinten über die Ringwulst 80 überstehenden Teil der Hülse 74 eine Sicherung 81 in Form einer Mutter 82 aufgeschraubt, um die Sicherung 81 zu bilden, die ein Herausfallen der Hülse 74 aus dem zweiten Teil 26 in Richtung des Pfeils 46 verhindert. Die Mutter 82 kann ferner dazu verwendet werden, um die Vorspannung der Druckfedern 84, 84' bzw. die Position der Hülse 74 in dem zweiten Teil 26 zu regulieren.

In einem zweiten Schritt werden nun die Kugeln 50, 50' in die Taschen 52, 52' eingeführt und die Hülse 60 entgegen der Richtung des Pfeils 46 von außen über das zweite Teil 26 aufgeschoben. In den zwischen der inneren Mantelfläche des zweiten Bereichs 63 der Hülse 60 und der äußeren Mantelfläche des zweiten Teils 26 gebildeten Raum werden die Druckfedern 64, 64' eingebracht. Abschließend wird nun durch Aufschieben in gleiche Richtung wie die Hülse 60 auf das zweite Teil 26 der dem zweiten Kupplungsteil 14 zugewandte Abschnitt des ersten Teils 22 auf den dem zweiten Kupplungsteil 14 abgewandten Teil des zweiten Teils 26 aufgeschoben, wobei das Innengewinde 28 des ersten Teils 22 mit dem Außengewinde 30 des zweiten Teils 26 verschraubt wird. Auf diese Weise wird nun das fertige erste Kupplungsteil 12 erhalten, das zum Beispiel durch Verschrauben in einem weiteren Schritt mit einem Saug- und/oder Spülschlauch oder auch direkt mit einem medizinischen Instrument verbunden werden kann.

Ein Auseinanderbauen des ersten Kupplungsteils 12 ist durch Durchführen der genannten Arbeitsschritte in umgekehrter Reihenfolge ebenfalls einfach möglich, was die Reinigung und Wartung solcher Kupplungen stark vereinfacht.

Die Kupplung 10 ist insgesamt vorzugsweise dampfsterilisierbar, und zwar auch im zusammengesetzten Zustand des ersten Kupplungsteils 12. Des Weiteren ist die Kupplung 10 vorzugsweise insgesamt aus biokompatiblen Materialien aufgebaut.

## Patentansprüche

1. Kupplung für ein medizinisches Instrument zum Verbinden zweier Leitungen, mit einem ersten Kupplungsteil (12) und einem zweiten Kupplungsteil (14), das in das erste Kupplungsteil (12) in Längsrichtung der Kupplungsteile (12, 14) einführbar ist, weiterhin mit am ersten Kupplungsteil (12) angeordneten ersten Verriegelungsmitteln (48) und am zweiten Kupplungsteil (14) angeordneten zweiten Verriegelungsmitteln (54), wobei die ersten Verriegelungsmittel (48) zwischen einer Verriegelungsstellung, in der sie mit den zweiten Verriegelungsmitteln (54) in Eingriff stehen, und einer Entriegelungsstellung, in der sie mit den zweiten Verriegelungsmitteln (54) außer Eingriff stehen, beweglich sind, und ferner mit am ersten Kupplungsteil (12) angeordneten Arretiermitteln (58), die zwischen einer Arretierstellung, in der sie die ersten und die zweiten Verriegelungsmittel (48, 54) in Eingriff miteinander halten, und einer Entarretierstellung, in der die ersten und zweiten Verriegelungsmittel (48, 54) außer Eingriff bringbar sind, beweglich sind, **dadurch gekennzeichnet, dass** im ersten Kupplungsteil (12) ein in dessen Längsrichtung beweglicher Schieber (72) angeordnet ist, der beim Einführen des zweiten Kupplungsteils (14) in das erste Kupplungsteil (12) von einer vorgeschobenen in eine zurückgezogene Stellung bewegt wird, dass der Schieber (72) in die vorgeschobene Stellung vorgespannt ist und dass an einem dem ersten Kupplungsteil (12) zugewandten Ende (38) des zweiten Kupplungsteils (14) Dichtungsmittel (45) angeordnet sind, die in einem Zustand in dem das erste Kupplungsteil (12) mit dem zweiten Kupplungsteil (14) verbunden ist einen Innenraum der Kupplung gegenüber einem Fluid abdichten.

2. Kupplung nach Anspruch 1, **dadurch gekennzeichnet, dass** der Schieber (72) in der vorgeschobenen Stellung die ersten Verriegelungsmittel (48) in der Entriegelungsstellung hält, und dass diese dabei die Arretiermittel (58) in der Entarretierstellung halten.

3. Kupplung nach Anspruch 1 oder 2, **dadurch gekennzeichnet, dass** der Schieber (72) in der zurückgezogenen Stellung die ersten Verriegelungsmittel (48) freigibt, damit diese mit den zweiten Verriegelungsmitteln (54) in Eingriff kommen können, wonach die Arretiermittel (58) von der Entarretierstellung in die Arretierstellung bewegbar sind.

4. Kupplung nach einem der Ansprüche 1 bis 3, **dadurch gekennzeichnet, dass** die Vorspannung des Schiebers (72) ausreichend bemessen ist, um das zweite Kupplungsteil (14) aus dem ersten Kupplungsteil (12) auszustoßen, wenn die Arretiermittel (58) von der Arretierstellung in die Entarretierstellung bewegt werden.

5. Kupplung nach einem der Ansprüche 1 bis 4, **dadurch gekennzeichnet, dass** die Arretiermittel (58) bei der Bewegung von der Entarretierstellung in die Arretierstellung die ersten Verriegelungsmittel (48) aus der Entriegelungsstellung in die Verriegelungsstellung bewegen.

6. Kupplung nach Anspruch 5, **dadurch gekennzeichnet, dass** die ersten Verriegelungsmittel (48) zumindest einen Formkörper, vorzugsweise zumindest eine Kugel (50, 50'), aufweisen, der in einer Wand des ersten Kupplungsteils (12) angeordnet ist, und dass die Arretiermittel (58) eine die Wand des ersten Kupplungsteils (12) umgebende Hülse (60) aufweisen, die einen ersten Abschnitt (66) größeren Innendurchmessers und einen zweiten Abschnitt (68) kleineren Innendurchmessers aufweist, wobei zwischen dem ersten und zweiten Abschnitt eine Anlaufschräge (70) vorhanden ist.

7. Kupplung nach einem der Ansprüche 1 bis 6, **dadurch gekennzeichnet, dass** die Arretiermittel (58) durch eine in Längsrichtung erfolgende Bewegung von der Arretier- in die Entarretierstellung bewegbar sind.

8. Kupplung nach Anspruch 6 und 7, **dadurch gekennzeichnet, dass** der erste und der zweite Abschnitt (66, 68) der Hülse (60) in Längsrichtung hintereinander angeordnet sind und sich die Anlaufschräge (70) in Längsrichtung erstreckt.

9. Kupplung nach einem der Ansprüche 1 bis 6, **dadurch gekennzeichnet, dass** die Arretiermittel (58) durch eine in Umfangsrichtung erfolgende Bewegung von der Arretier- in die Entarretierstellung bewegbar sind.

10. Kupplung nach Anspruch 6 und 9, **dadurch gekennzeichnet, dass** der erste und zweite Abschnitt (66, 68) der Hülse (60) in Umfangsrichtung hintereinander angeordnet sind und sich die Anlaufschräge (70) in Umfangsrichtung erstreckt.

11. Kupplung nach einem der Ansprüche 1 bis 10, **dadurch gekennzeichnet, dass** der Schieber (72) in Form einer innerhalb des ersten Kupplungsteils (12) angeordneten Hülse (74) ausgebildet ist, die an einer Innenseite des ersten Kupplungsteils (12) geführt ist, wobei ein Anschlag für die Hülse (74) in der vorgeschobenen Stellung vorgesehen ist.

12. Kupplung nach einem der Ansprüche 1 bis 11, **dadurch gekennzeichnet, dass** ein vorderes Ende des Schiebers (72) mit einer Anlaufschräge versehen ist.

13. Kupplung nach einem der Ansprüche 1 bis 12, **dadurch gekennzeichnet, dass** die Dichtungsmittel (45) einen O-Ring (44) aufweisen, der in einer Ringnut (42) in dem dem ersten Kupplungsteil (12) zugewandten Ende des zweiten Kupplungsteils (14) angeordnet ist und der in einem Zustand in dem das erste Kupplungsteil (12) mit dem zweiten Kupplungsteil (14) verbunden ist gegen eine Innenseite einer Bohrung in dem ersten Kupplungsteil (12) anliegt.

14. Verfahren zum Herstellen eines ersten Kupplungsteils (12) einer Kupplung (10) für ein medizinisches Instrument zum Verbinden zweier Leitungen, mit den folgenden Schritten, nämlich
Bereitstellen eines zweiten Teils (26) eines Körpers (20) des ersten Kupplungsteils (12), wobei das zweite Teil (26) röhrenförmig ausgebildet ist, und wobei dieses an seiner inneren Mantelfläche zumindest einen ersten Vorsprung, und an einem dem zweiten Kupplungsteil (14) zugewandten Ende an der äußeren Mantelfläche zumindest einen zweiten Vorsprung aufweist,
Einführen einer inneren Hülse (74) in das zweite Teil (26), wobei zwischen einem ersten Abschnitt (76) der Hülse (74) und dem ersten Vorsprung des zweiten Teils (26) elastische Elemente vorgesehen sind, die auf die Hülse (74) eine Kraft entgegen der Richtung des Einführens ausüben,
Anbringen einer Sicherung (81) an der inneren Hülse (74) wobei die Sicherung (81) in Richtung des Einführen stromabwärts des ersten Vorsprungs des zweiten Teils (26) an der Hülse (74) angebracht wird um ein Herausfallen der Hülse aus dem zweiten Teil (26) zu verhindern,
Bereitstellen von Verriegelungsmitteln (48) in dem zweiten Teil (26),
Aufschieben einer äußeren Hülse (60) auf das zweite Teil (26), wobei die äußere Hülse (60) einen ersten Bereich (62) mit einem kleineren Innendurchmesser und einen zweiten Bereich (63) mit einem größeren Innendurchmesser aufweist,
Verbinden eines ersten Teils (22) des Körpers mit dem zweiten Teil (26), wobei das erste Teil (22) die Bewegung der äußeren Hülse (60) in einer Richtung entgegen der Richtung ihres Aufschiebens begrenzt und wobei in dem Bereich des zweiten Abschnitts (63) zwischen der äußeren Hülse (60) und dem ersten Teil (22) elastische Elemente vorgesehen sind, die auf die äußere Hülse ein Kraft in Richtung des Aufschiebens ausüben.

15. Verfahren nach Anspruch 14, **dadurch gekennzeichnet, dass** die elastischen Elemente zumindest eine Druckfeder (64, 64', 84, 84') aufweisen.

16. Verfahren nach Anspruch 14 oder 15, **dadurch gekennzeichnet, dass** die Verrieglungsmittel (48) zumindest eine Kugel (50, 50') aufweisen, die in einer Richtung quer zur Längsachse des ersten Kupplungsteils (12) verschiebbar ist.

17. Verfahren nach einem der Ansprüche 14 bis 16, **dadurch gekennzeichnet, dass** der erste Vorsprung eine Ringwulst (80) ist.

18. Verfahren nach einem der Ansprüche 14 bis 17, **dadurch gekennzeichnet, dass** der zweite Vorsprung ein Ringflansch ist.

19. Verfahren nach einem der Ansprüche 14 bis 18, **dadurch gekennzeichnet, dass** die Sicherung (81) eine Mutter (82) ist, die auf die innere Hülse (74) aufgeschraubt wird.

20. Verfahren nach einem der Ansprüche 14 bis 18, **dadurch gekennzeichnet, dass** die Sicherung (81) und die innere Hülse (74) einstückig ausgebildet sind.

## Claims

1. Coupling for a medical instrument for connecting two lines, with a first coupling part (12), and with a second coupling part (14) which can be inserted into the first coupling part (12) in the longitudinal direction of the coupling parts (12, 14), with first locking elements (48) arranged on the first coupling part (12) and second locking elements (54) arranged on the second coupling part (14), the first locking elements (48) being movable between a locking position, in which they engage with the second locking elements (54), and an unlocking position, in which they disengage from the second locking elements (54), and also with retaining elements (58) which are arranged on the first coupling part (12) and which are movable between a retention position, in which they keep the first and second locking elements (48, 54) in engagement with each other, and a release position, in which the first and second locking elements (48, 54) can be disengaged, **characterized in that** the first coupling part (12) comprises a slide (72) which is movable in the longitudinal direction thereof and which, upon insertion of the second coupling part (14) into the first coupling part (12), is moved from an advanced position to a withdrawn position, **in that** the slide (72) is biased into the advanced position, and **in that** sealing elements (45) are arranged on an end (38) of the second coupling part (14) directed towards the first coupling part (12) which in a state in which the first coupling part (12) is connected to the second coupling part (14), seal off an interior of the coupling with respect to a fluid.

2. Coupling according to Claim 1, **characterized in that** the slide (72), in the advanced position, keeps the first locking elements (48) in the unlocking position, and **in that** these keep the retaining elements (58) in the release position.

3. Coupling according to Claim 1 or 2, **characterized in that** the slide (72), in the withdrawn position, frees the first locking elements (48), such that these can come into engagement with the second locking elements (54), after which the retaining elements (58) are movable from the release position to the retention position.

4. Coupling according to any one of Claims 1 to 3, **characterized in that** the bias of the slide (72) is sufficient to eject the first coupling part (12) from the second coupling part (14) when the retaining elements (58) are moved from the retention position to the release position.

5. Coupling according to any one of Claims 1 to 4, **characterized in that** the retaining elements (58), during the movement from the release position to the retention position, move the first locking elements (48) from the unlocking position to the locking position.

6. Coupling according to Claim 5, **characterized in that** the first locking elements (48) comprise at least one shaped body, preferably at least one ball (50, 50'), which is arranged in a wall of the first coupling part (12), and **in that** the retaining elements (58) comprise a sleeve (60) which surrounds the wall of the first coupling part (12) and which comprises a first section (66) having a larger internal diameter and a second section (68) having a smaller diameter, with a run-on slope (70) being present between the first and second sections.

7. Coupling according to any one of Claims 1 to 6, **characterized in that** the retaining elements (58) are movable from the retention position to the release position by a movement that takes place in the longitudinal direction.

8. Coupling according to Claims 6 and 7, **characterized in that** the first and second sections (66, 68) of the sleeve (60) are arranged one after the other in the longitudinal direction, and that the run-on slope (70) extends in the longitudinal direction.

9. Coupling according to any one of Claims 1 to 6, **characterized in that** the retaining elements (58) are movable from the retention position to the release position by a movement that takes place in the circumferential direction.

10. Coupling according to Claims 6 and 9, **characterized in that** the first and the second section (66, 68) of the sleeve (60) are arranged one after the other in the circumferential direction, and that the run-on slope (70) extends in the circumferential direction.

11. Coupling according to any one of Claims 1 to 10, **characterized in that** the slide (72) is designed in the form of a sleeve (74) which is arranged inside the first coupling part (12) and which is guided on an inner face of the first coupling part (12), with an abutment being provided for the sleeve in the advanced position.

12. Coupling according to any one of Claims 1 to 11, **characterized in that** a front end of the slide (72) is provided with a run-on slope.

13. Coupling according to any one of Claims 1 to 12, **characterized in that** the sealing elements (45) comprise an O-ring (44) which is arranged in an annular groove (42) in the end of the second coupling part (14) directed towards the first coupling part (12) and which, in a state when the first coupling part (12) is connected to the second coupling part (14), bears against an inner face of a bore in the first coupling part (12).

14. Method for producing a first coupling part (12) of a coupling (10) for a medical instrument for connecting two lines, said method comprising the following steps:
providing a second part (26) of a body (20) of the first coupling part (12), which second part (26) is tubular and comprises at least one first projection on its inner circumferential surface and, at an end directed towards the second coupling part (14), comprises at least one second projection on the outer circumferential surface,
inserting an inner sleeve (74) into the second part (26), wherein elastic elements are provided between a first section (76) of the sleeve (74) and the first projection of the second part (26), which elastic elements subject the sleeve (74) to a force counter to the direction of insertion,
arranging a safety device (81) on the inner sleeve (74), wherein the safety device (81) is mounted on the sleeve (74) at a position downstream of the first projection of the second part (26) in the direction of insertion, in order to prevent the sleeve falling out of the second part (26),
providing locking elements (48) in the second part (26),
pushing an outer sleeve (60) onto the second part (26), wherein the outer sleeve (60) comprises a first section (62) having a smaller internal diameter and a second section (63) having a larger internal diameter,
connecting a first part (22) of the body to the second part (26), wherein the first part (22) limits the movement of the outer sleeve (60) in a direction counter to the direction in which it is pushed on, and wherein elastic elements are provided in the area of the second section (63) between the outer sleeve (60) and the first part (22), which elastic elements subject the outer sleeve to a force acting in the direction in which it is pushed on.

15. Method according to Claim 14, **characterized in that** the elastic elements comprise at least one compression spring (64, 64' 84, 84').

16. Method according to Claim 14 or 15, **characterized in that** the locking elements (48) comprise at least one ball (50, 50') which is displaceable in a direction transverse to the longitudinal axis of the first coupling part (12).

17. Method according to any one of Claims 14 to 16, **characterized in that** the first projection is an annular bead (80).

18. Method according to any one of Claims 14 to 17, **characterized in that** the second projection is an annular flange.

19. Method according to any one of Claims 14 to 18, **characterized in that** the safety device (81) is a nut (82) which is screwed onto the inner sleeve (74).

20. Method according to any one of Claims 14 to 18, **characterized in that** the safety device (81) and the inner sleeve (74) are formed in one piece.

## Revendications

1. Raccord pour un instrument médical pour relier deux conduites, comprenant une première partie de raccord (12) et une deuxième partie de raccord (14) qui peut être insérée dans la première partie de raccord (12) dans la direction longitudinale des parties de raccord (12, 14), comprenant en outre des premiers moyens de verrouillage (48) disposés sur la première partie de raccord (12) et des deuxièmes moyens de verrouillage (54) disposés sur la deuxième partie de raccord (14), les premiers moyens de verrouillage (48) étant mobiles entre une position de verrouillage, dans laquelle ils sont en prise avec les deuxièmes moyens de verrouillage (54), et une position de déverrouillage, dans laquelle ils ne sont pas en prise avec les deuxièmes moyens de verrouillage (54), et comprenant de plus des moyens de blocage (58) disposés sur la première partie de raccord (12) qui sont mobiles entre une position de blocage, dans laquelle ils maintiennent les premiers et deuxièmes moyens de verrouillage (48, 54) en prise les uns avec les autres, et une position de déblocage, dans laquelle les premiers et deuxièmes moyens de verrouillage (48, 54) peuvent être amenés hors de prise, **caractérisé en ce que**, dans la première partie de raccord (12), est disposé un coulisseau (72) mobile dans la direction longitudinale de celle-ci, lequel coulisseau est déplacé à partir d'une position avancée jusqu'à une position rétractée lors de l'insertion de la deuxième partie de raccord (14) dans la première partie de raccord (12), **en ce que** le coulisseau (72) est précontraint dans la position avancée et **en ce que** des moyens d'étanchéité (45) sont disposés à une extrémité (38) de la deuxième partie de raccord (14) tournée vers la première partie de raccord (12), lesquels réalisent l'étanchéité d'un espace intérieur du raccord par rapport à un fluide dans un état dans lequel la première partie de raccord (12) est reliée à la deuxième partie de raccord (14).

2. Raccord selon la revendication 1, **caractérisé en ce que**, dans la position avancée, le coulisseau (72) maintient les premiers moyens de verrouillage (48) dans la position de déverrouillage, et **en ce que** ceux-ci maintiennent en l'occurrence les moyens de blocage (58) dans la position de déblocage.

3. Raccord selon la revendication 1 ou 2, **caractérisé en ce que**, dans la position rétractée, le coulisseau (72) libère les premiers moyens de verrouillage (48) afin que ceux-ci puissent venir en prise avec les deuxièmes moyens de verrouillage (54), après quoi les moyens de blocage (58) peuvent être déplacés à partir de la position de déblocage jusqu'à la position de blocage.

4. Raccord selon l'une quelconque des revendications 1 à 3, **caractérisé en ce que** la précontrainte du coulisseau (72) est évaluée de manière à être suffisante pour éjecter la deuxième partie de raccord (14) hors de la première partie de raccord (12) lorsque les moyens de blocage (58) sont déplacés à partir de la position de blocage jusqu'à la position de déblocage.

5. Raccord selon l'une quelconque des revendications 1 à 4, **caractérisé en ce que** les moyens de blocage (58) déplacent les premiers moyens de verrouillage (48) à partir de la position de déverrouillage jusqu'à la position de verrouillage lors du déplacement à partir de la position de déblocage jusqu'à la position de blocage.

6. Raccord selon la revendication 5, **caractérisé en ce que** les premiers moyens de verrouillage (48) comprennent au moins un corps moulé, de préférence au moins une bille (50, 50'), qui est disposé dans une paroi de la première partie de raccord (12), et **en ce que** les moyens de blocage (58) comprennent une douille (60) entourant la paroi de la première partie de raccord (12), laquelle douille comprend une première section (66) de plus grand diamètre intérieur et une deuxième section (68) de plus petit diamètre intérieur, un biseau d'introduction (70) étant présent entre la première et la deuxième section.

7. Raccord selon l'une quelconque des revendications 1 à 6, **caractérisé en ce que** les moyens de blocage (58) peuvent être déplacés à partir de la position de blocage jusqu'à la position de déblocage par un déplacement s'effectuant dans la direction longitudinale.

8. Raccord selon les revendications 6 et 7, **caractérisé en ce que** les première et deuxième sections (66, 68) de la douille (60) sont disposées l'une derrière l'autre dans la direction longitudinale et **en ce que** le biseau d'introduction (70) s'étend dans la direction longitudinale.

9. Raccord selon l'une quelconque des revendications 1 à 6, **caractérisé en ce que** les moyens de blocage (58) peuvent être déplacés à partir de la position de blocage jusqu'à la position de déblocage par un déplacement s'effectuant dans la direction périphérique.

10. Raccord selon les revendications 6 et 9, **caractérisé en ce que** les première et deuxième sections (66, 68) de la douille (60) sont disposées l'une derrière l'autre dans la direction périphérique et **en ce que** le biseau d'introduction (70) s'étend dans la direction périphérique.

11. Raccord selon l'une quelconque des revendications 1 à 10, **caractérisé en ce que** le coulisseau (72) est réalisé sous la forme d'une douille (74) disposée à l'intérieur de la première partie de raccord (12), laquelle douille est guidée contre un côté intérieur de la première partie de raccord (12), une butée pour la douille (74) dans la position avancée étant prévue.

12. Raccord selon l'une quelconque des revendications 1 à 11, **caractérisé en ce qu'**une extrémité avant du coulisseau (72) est pourvue d'un biseau d'introduction.

13. Raccord selon l'une quelconque des revendications 1 à 12, **caractérisé en ce que** les moyens d'étanchéité (45) comprennent un joint torique (44) qui est disposé dans une rainure annulaire (42) dans l'extrémité de la deuxième partie de raccord (14) tournée vers la première partie de raccord (12) et qui, dans un état dans lequel la première partie de raccord (12) est reliée à la deuxième partie de raccord (14), s'applique contre un côté intérieur d'un alésage dans la première partie de raccord (12).

14. Procédé pour fabriquer une première partie de raccord (12) d'un raccord (10) pour un instrument médical pour relier deux conduites, comprenant les étapes suivantes, à savoir
la fourniture d'une deuxième partie (26) d'un corps (20) de la première partie de raccord (12), la deuxième partie (26) étant tubulaire, et celle-ci comprenant, sur sa surface d'enveloppe intérieure, au moins une première saillie et, à une extrémité tournée vers la deuxième partie de raccord (14) sur la surface d'enveloppe extérieure, au moins une deuxième saillie,
l'insertion d'une douille intérieure (74) dans la deuxième partie (26), des éléments élastiques étant prévus entre une première section (76) de la douille (74) et la première saillie de la deuxième partie (26), lesquels exercent sur la douille (74) une force en sens contraire au sens d'insertion,
installation d'un dispositif de fixation (81) sur la douille intérieure (74), le dispositif de fixation (81) étant installé en aval de la première saillie de la deuxième partie (26) sur la douille (74) dans le sens d'insertion, afin d'empêcher que la douille ne tombe hors de la deuxième partie (26),
fourniture de moyens de verrouillage (48) dans la deuxième partie (26),
enfilage d'une douille extérieure (60) sur la deuxième partie (26), la douille extérieure (60) comprenant une première région (62) présentant un diamètre intérieur plus petit et une deuxième région (63) présentant un diamètre intérieur plus grand,
liaison d'une première partie (22) du corps à la deuxième partie (26), la première partie (22) limitant le déplacement de la douille extérieure (60) dans un sens opposé à son sens d'enfilage, et des éléments élastiques étant prévus entre la douille extérieure (60) et la première partie (22) dans la région de la deuxième section (63), lesquels exercent sur la douille extérieure une force dans le sens de l'enfilage.

15. Procédé selon la revendication 14, **caractérisé en ce que** les éléments élastiques comprennent au moins un ressort de compression (64, 64', 84, 84').

16. Procédé selon la revendication 14 ou 15, **caractérisé en ce que** les moyens de verrouillage (48) comprennent au moins une bille (50, 50') qui peut être déplacée dans une direction transversale par rapport à l'axe longitudinal de la première partie de raccord (12).

17. Procédé selon l'une quelconque des revendications 14 à 16, **caractérisé en ce que** la première saillie est un bourrelet annulaire (80).

18. Procédé selon l'une quelconque des revendications 14 à 17, **caractérisé en ce que** la deuxième saillie est un rebord annulaire.

19. Procédé selon l'une quelconque des revendications 14 à 18, **caractérisé en ce que** le dispositif de fixation (81) est un écrou (82) qui est vissé sur la douille intérieure (74).

20. Procédé selon l'une quelconque des revendications 14 à 18, **caractérisé en ce que** le dispositif de fixation (81) et la douille intérieure (74) sont réalisés d'une seule pièce.
